# EUROPEAN PATENT APPLICATION

(11) **EP 4 789 635 A1**
(43) Date of publication of application: **12.08.2026**
(21) Application number: 25157072.7
(22) Date of filing: 11.02.2025
(51) Int. Cl.: A61B 34/20, A61F 2/44, A61B 17/16, A61B 17/88

(54) **TECHNIQUE FOR ASSESSING A RELATIVE POSE BETWEEN A SURGICAL INSTRUMENT AND A FUNCTIONAL ELEMENT**

(71) Applicant: Stryker European Operations Limited, Carrigtwohill, Co. Cork T45 HX08 (IE)
(72) Inventor: Umbdenstock, Emeric, 00185 Roma (IT); Xia, Yan, 01099 Dresden (DE); Herrmann, Florian, 77963 Schwanau (DE); Koepff, Marvin, 79227 Schallstadt (DE); Sirkin, Max, 79098 Freiburg (DE)
(74) Representative: Schott, Jakob Valentin

(57) **Abstract**

Disclosed is a method for supporting a surgeon in assessing a relative pose between a surgical instrument and a functional element configured to be coupled thereto. The method may comprise obtaining an image of the functional element, determining a tracked pose of the functional element, obtaining a tracked pose of the surgical instrument, determining a relative pose between the functional element and the surgical instrument, and triggering output of feedback for a surgeon, the feedback indicating whether the relative pose meets one or more predefined criteria. A surgical navigation, system a computer program and a carrier are also disclosed.

## Description

### Technical Field

The present disclosure generally relates to a method for supporting a surgeon in assessing a relative pose between a surgical instrument and a functional element configured to be coupled thereto. A surgical navigation, system a computer program and a carrier are also disclosed.

### Background

In surgical procedures, surgical instruments are often used to position functional elements such as implants or tool tips relative to a patient's body. Examples of implants include bone nails, bone screws (e.g., pedicle screws), bone plates and spinal implants such as interbody implants or spinal rods. Examples of tool tips include a saw blade, a chisel tip and a drill attachment. In case the functional element to be positioned is a bone screw, the surgical instrument may be a surgical screwdriver. In case the functional element to be positioned is a spinal cage, the surgical instrument may be a cage inserter. In case the functional element to be positioned is a saw blade, the surgical instrument may be a surgical saw. Other combinations of functional elements and surgical elements may be apparent to those skilled in the art.

In some scenarios, it may be desired to place the functional element at a desired pose relative to the patient's body. To this end, the surgical instrument used to position the functional element may be tracked by a tracking system. The tracked pose of the surgical instrument may be used to derive a predicted pose of the functional element coupled to the surgical instrument.

This approach has the drawback that the predicted pose of the functional element may not be correct, especially if the functional element was coupled to the surgical instrument in an erroneous manner. A surgeon using the surgical instrument may thus need to visually check correct coupling of the functional element to the surgical element. Such a check is cumbersome for the surgeon and may not be reliable.

### Summary

There is a need for a technique that solves one or more of the aforementioned or other problems.

According to a first aspect, a method for supporting a surgeon in assessing a relative pose between a surgical instrument and a functional element configured to be coupled thereto is provided. The method comprises: obtaining tracking image data comprising at least one image of at least a portion of a functional element that is configured to be coupled to a surgical instrument; determining, based on the tracking image data, optionally via object recognition, a tracked pose of the functional element; obtaining a tracked pose of the surgical instrument; determining, based on the tracked pose of the functional element and the tracked pose of the surgical instrument, a relative pose between the functional element and the surgical instrument; and triggering output of feedback for a surgeon, the feedback indicating whether the relative pose meets one or more predefined criteria.

The method may be performed by a surgical navigation system. The method may be referred to as a computer-implemented method. The method may be a non-surgical method, i.e., may not comprise a surgical step, in particular not comprise any step that requires a substantial interaction with the body of a human or animal patient. In one particular variant, the tracked pose of the functional element is determined based on the tracking image data without object recognition.

Unless indicated otherwise, the term "pose" as used herein shall refer to at least one of a position and an orientation, preferably a combination of both, wherein the pose may be defined in three dimensions and/or six degrees of freedom.

The tracked pose of the functional element may be indicative of or correspond to a pose of an axis of the functional element. The tracked pose of the surgical instrument may be indicative of or correspond to a pose of an axis of the surgical instrument. The one or more predefined criteria may comprise a maximum allowable angle between these two axes.

The tracking image data may comprise at least one image of {i} at least a portion of the surgical instrument and/or {ii} an attachment rigidly attached to the surgical instrument. For example, the method further comprises determining, based on the tracking image data, optionally via object recognition, the tracked pose of the surgical instrument.

For example, the at least one image comprises an image acquired with a camera configured to detect light having wavelengths perceptible by a human eye, such as an RGB image. The tracked pose of the functional element and the tracked pose of the surgical instrument may be determined based on data obtained from a tracking unit comprising at least one near-infrared, NIR, camera. For example, the tracking image data is obtained from the tracking unit comprising the camera configured to detect light having wavelengths perceptible by a human eye.

The method may further comprise determining a deviation between: {i} the determined relative pose between the functional element and the surgical instrument; and {ii} an ideal relative pose between the functional element and the surgical instrument, optionally derived from a predefined three-dimensional ideal model. The one or more predefined criteria may comprise a maximum allowable deviation.

The tracking image data may comprise a plurality of two-dimensional images of at least the portion of the functional element that have each been acquired at different points in time. The tracked pose of the functional element or a pose of an axis of the functional element at one of these points in time may be determined in three dimensions based on said plurality of images. Alternatively, or in addition, a length of the functional element may be determined based on said plurality of images.

One or more possible two-dimensional poses of at least the portion of the functional element may be determined for each of the plurality of two-dimensional images. The tracked pose of the functional element or a pose of an axis of the functional element, at one of the different points in time, may be determined in three dimensions based on said one or more possible two-dimensional poses. Alternatively, or in addition, a (e.g., the) length of the functional element may be determined based on said one or more possible two-dimensional poses.

In one example, the method further comprises determining (e.g., based on the tracked pose of the instrument) a spatial region in which at least one object of interest is expected to be located. The method may comprise selecting a region in each of one or more of the at least one image comprised in the tracking image data that corresponds to the spatial region. The method may comprise detecting the at least one object of interest in the selected region(s). The at least one object of interest may comprise the functional element and/or the surgical instrument.

For example, the feedback is triggered to be output if the relative pose is indicative of the functional element being coupled to the surgical instrument. Alternatively, or in addition, the one or more predefined criteria may require the functional element to be coupled to the surgical instrument.

The method may further comprise obtaining patient tracking data indicative of a pose of a patient's body. The method may comprise determining, based the patient tracking data and the determined pose of the functional element, a distance between the functional element and the patient's body. The feedback may be triggered to be output if the distance is smaller than a predefined maximum distance. Alternatively, or in addition, the one or more predefined criteria may require the distance to be smaller than a predefined maximum distance.

In one example, the one or more predefined criteria are specific for the functional element and/or the surgical instrument and/or a combination of the functional element with the surgical instrument.

In one example, the functional element is a bone screw such as a pedicle screw and the surgical instrument is a screwdriver. In another example, the functional element is an implant such as an interbody implant (e.g., a spinal cage) and the surgical instrument is an implant inserter (e.g., a cage inserter). In another example, the functional element is a material removal unit such as a saw, a drill or a burr and the surgical instrument is a power tool configured to operate the functional element.

According to a second aspect, a surgical navigation system is provided. The surgical navigation system comprises at least one processor configured to perform the method of the first aspect. The surgical navigation system may further comprise a tracking unit configured to acquire one of more images and/or a feedback unit such as a display configured to provide the feedback to a user and/or the functional element and/or the surgical instrument.

According to a third aspect, a computer program is provided. The computer program comprises instructions which, when the program is executed by at least one processor (e.g., of the surgical navigation system of the second aspect), cause the at least one processor to carry out the method of the first aspect. The computer program may be carried by a carrier such as a data stream, a portable memory device or a non-transitory computer storage medium.

According to a fourth aspect, a carrier is provided. The carrier may be a data stream, a portable memory device or a non-transitory computer storage medium. The carrier carries the computer program of the third aspect.

### Brief Description of the Drawings

Further details, advantages and aspects of the present disclosure will become apparent from the following examples in conjunction with the drawings, wherein:
- Fig. 1: shows an exemplary surgical navigation system in accordance with the present disclosure;
- Fig. 2: shows an exemplary functional element coupled to an exemplary surgical instrument;
- Fig. 3: shows a first relative pose between an exemplary functional element and an exemplary surgical instrument;
- Fig. 4: shows a second relative pose between an exemplary functional element and an exemplary surgical instrument; and

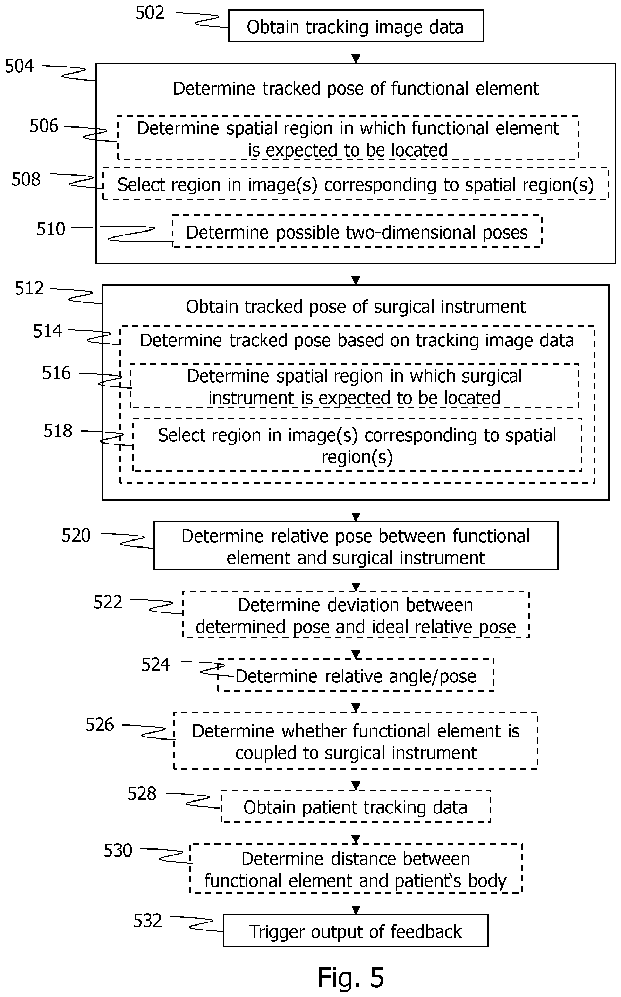

- Fig. 5: shows a flowchart of a method in accordance with the present disclosure.

### Detailed Description

In the following description, exemplary embodiments will be explained with reference to the drawings. Unless indicated otherwise, the reference signs used in the following denote the same or similar structural or functional features. In case an example shows more than one instance of a given entity, which entity is denoted with reference numeral "X", these instances may be referred to either as "X", or as "X-n" with n indicating the particular instance.

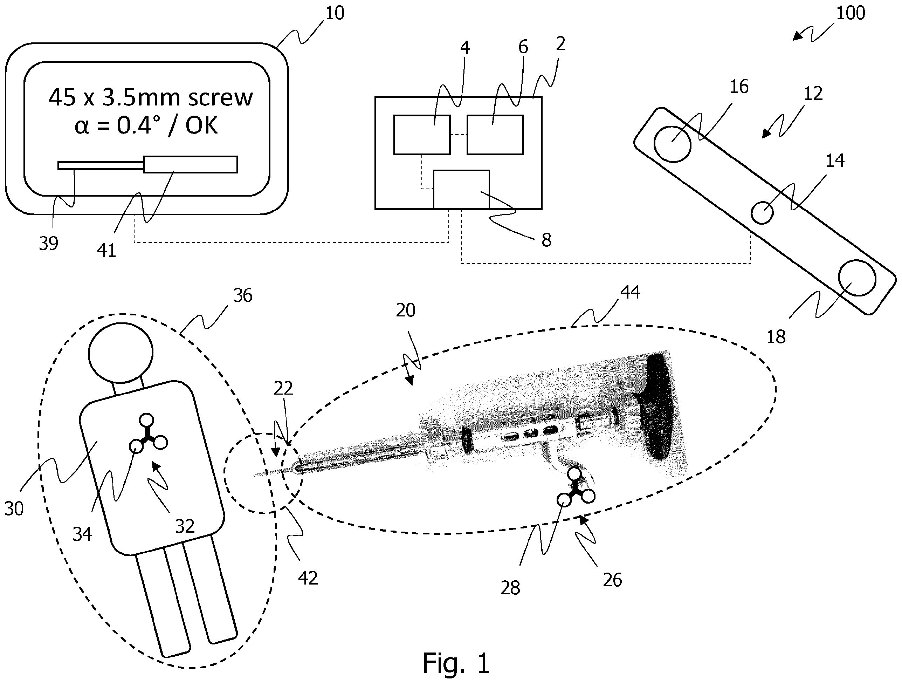

Fig. 1 shows an exemplary surgical navigation system 100. The system 100 comprises at least one processor 4 that is communicatively coupled to at least one memory 6. The at least one memory 6 stores instructions that, when executed by the at least one processor 4, cause the at least one processor 4 to perform the method disclosed herein.

The at least one processor 4 and the at least one memory 6 may be part of one and the same processing unit 2, although a distributed computing environment is also envisaged. The processing unit 2 comprises at least one interface 8 that is communicatively coupled to a feedback unit such as a display 10, which is configured to provide feedback to a user. The at least one interface 8 is further communicatively coupled to a tracking unit 12 to receive data from said tracking unit 12. The tracking unit 12 and the feedback unit 10 may be part of the surgical navigation system 100.

The tracking unit 12 can be an optical tracking unit comprising two infrared cameras 16, 18. The tracking unit 12 may further comprise an RGB camera 14. A relative pose of the fields of view of the cameras 14, 16, 18 may be known to the at least one processor 4.

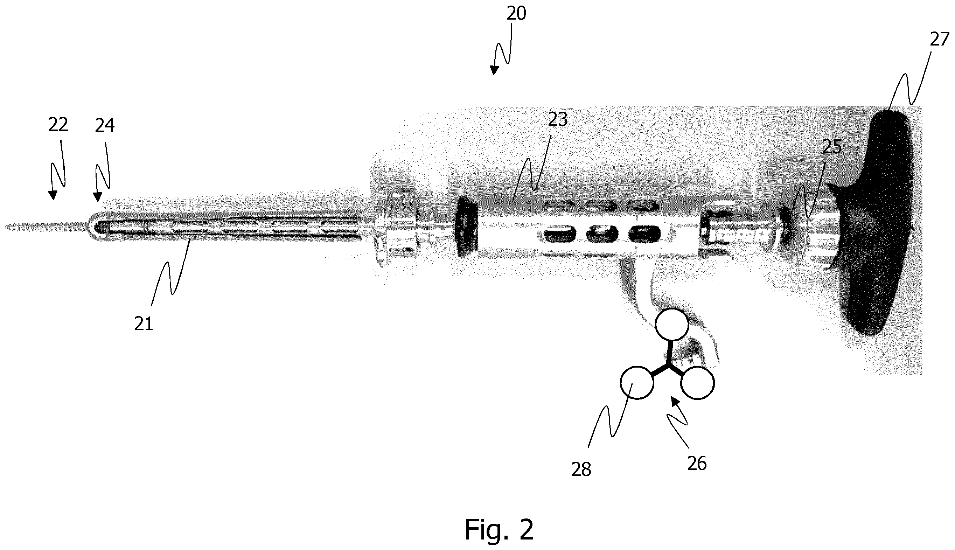

Fig. 1 also shows a surgical instrument 20 having a functional element 22 coupled thereto. In this illustrated example, and as also apparent from the enlarged view shown in Fig. 2, the surgical instrument 20 is a surgical screwdriver and the functional element 22 is a pedicle screw coupled to a distal end 24 of said screwdriver. The screwdriver can have a first sleeve 21 extending proximally from the distal end 24 and a second sleeve 23 arranged axially offset thereto in the proximal direction. A torque transmitting member 25 can extend through these sleeves 21, 23 and end in the distal end 24, thereby being configured to transmit torque from a handle 27 to the pedicle screw. Other types of surgical screwdrivers are known to those skilled in the art.

A tracker 26 comprising a plurality of optical tracking markers 28 (e.g., reflective tracking markers and/or active light-emitting tracking markers) is attached in a known pose relative to the surgical instrument 20. The optical tracking markers 28 can be localized by the optical tracking unit 12. Based on the known pose of the tracker 26 relative to the surgical instrument 20, the pose of the surgical instrument 20 can be determined in three dimensions once the pose of the tracker 26 has been determined by localizing its optical tracking markers 28. Fig. 1 also schematically illustrates a patient 30 that may be located on a patient couch within an operating room. An optical patient tracker 32 may be arranged in a fixed spatial relationship relative to the patient (e.g., attached to the patient or the patient couch). The optical patient tracker 32 may comprise a plurality of optical tracking markers 34 (e.g., reflective tracking markers and/or active light-emitting tracking markers) that can be localized by the optical tracking unit 12. A virtual boundary 36 can be defined relative to the patient 30, for example relative to the pose of the optical patient tracker 32. It is noted that other (e.g., non-optical) tracking techniques (e.g., not requiring the optical tracker 26 and/or 32) could be used instead. A spatial region in which the pedicle screw is expected to be located is indicated with reference sign 42. A spatial region in which the screwdriver is expected to be located is indicated with reference sign 44.

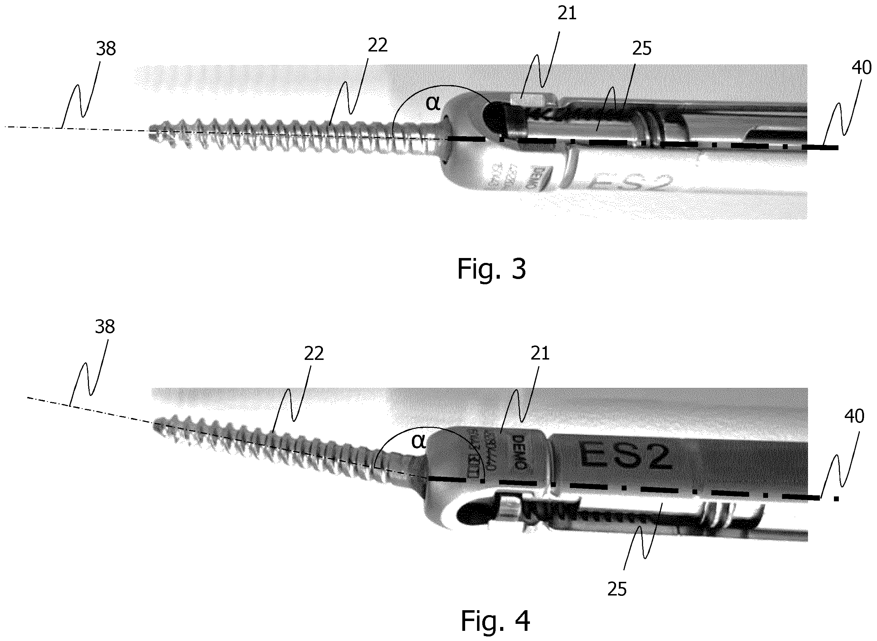

Fig. 3 shows a first relative pose between the pedicle screw and the screwdriver of Fig. 1 and Fig. 2. In this case, a longitudinal axis 38 of the pedicle screw is aligned with a longitudinal axis 40 of the screwdriver. The longitudinal axis 40 of the screwdriver in the illustrated example corresponds to a central axis of the sleeve 21 and a rotational axis of the torque transmitting member 25. As the two axes 38, 40 are aligned perfectly, a relative angle α between these two axes 38, 40 corresponds to 180°.

Fig. 4 shows a second relative pose between the pedicle screw and the screwdriver of Fig. 1 and Fig. 2. In this case, the longitudinal axis 38 of the pedicle screw is misaligned with the longitudinal axis 40 of the screwdriver. In the illustrated example, a relative angle α between these two misaligned axes 38, 40 corresponds to 165°. Such a misalignment may not be acceptable, as it could lead to a comparatively large mispositioning of the pedicle screw relative to the patient 30 in case the screw is navigated based on a tracked pose of the surgical instrument derived from the pose of the tracker 26.

It is to be understood that these particular examples are not limiting. In some scenarios, a given angle α that differs from 180° may be preferred, for example in case the trajectory for inserting the screw into the pedicle differs from an insertion trajectory of the sleeve 21 within the patient's body. Thus, the acceptable angle α may differ from the surgical scenario at hand. In any case, the preferable angle may be known to the at least one processor 4, either by being input (e.g., by a user) or derived (e.g., from a surgical plan). As a default, an angle α of 180° may be assumed to represent a preferred coupling between the pedicle screw and the screwdriver. The relative angle α may be different for other types of functional elements 22 and surgical instruments 20.

Fig. 3 shows a flowchart of an exemplary method in accordance with the present disclosure. While the reference signs of Figs. 1 to 4 will be used in the following, it is to be noted that the method is not limited to these particular examples. Accordingly, the reference signs 2 to 44 when used in the following should be understood as exemplary and may in one variant be omitted.

The method shall support a surgeon in assessing a relative pose between a surgical instrument 20 and a functional element 22 configured to be coupled thereto. The method may be performed by the at least one processor 4, although this is not essential. Optional aspects of the method are shown in dashed boxes in Fig. 3. It is to be understood that the sequence of the individual steps as shown in Fig. 3 may be changed and/or various steps may be combined with one another (e.g., as part of a common step). It is also possible to divide common steps into a plurality of individual steps.

The method comprises a step 502 of obtaining tracking image data. The tracking image data comprises at least one image of at least a portion of a functional element 22 that is configured to be coupled to a surgical instrument 20.

The tracking image data and/or the at least one image are preferably obtained from the tracking unit 12. The at least one image in a preferred example comprises an image acquired with a camera configured to detect light having wavelengths perceptible by a human eye, such as an RGB image. The at least one image may in particular comprise an image acquired by the camera 14. The tracking image data may comprise a plurality of (e.g., two-dimensional images) of at least the portion of the functional element 22 that have each been acquired at different points in time. The at least one image may be part of a (e.g., RGB) video stream captured by a camera such as the camera 14. In one particular example, the tracking image data does not comprise an infrared image, a stereo image and/or an image acquired by the camera 16 or 18. In this case, one may say that the tracking image data consists of images acquired by the camera 14 and/or consists of RBG images.

The functional element 22, at least a portion of which is depicted in the at least one image comprised in the tracking image data, may be a bone screw such as a pedicle screw and the surgical instrument 20 may be a screwdriver, as exemplarily shown in Figs. 1 to 4. Alternatively, the functional element 22 may be an implant such as an interbody implant and the surgical instrument 20 may be an implant inserter. As a still further alternative, the functional element 22 may be a material removal unit such as a saw, a drill or a burr and the surgical instrument 20 may be a power tool configured to operate the functional element 22.

The method comprises a step 504 of determining, based on the tracking image data, a tracked pose of the functional element. The tracked pose of the functional element 22 is in particular indicative of a pose of an axis 38 of the functional element 22. Said axis may correspond to an axis of symmetry, a longitudinal axis and/or an insertion axis of the functional element 22.

The tracked pose may be determined via object recognition (i.e., using at least one object recognition algorithm and/or computer program). The object recognition may yield the tracked pose of the functional element from the at least one image comprised in the tracking image data. Alternatively, or in addition, a contour detection, edge detection and/or object classification may be applied to the at least one image comprised in the tracking image data to at least determine the tracked pose of the functional element. This approach for determining the tracked pose of the functional element differs from the regular approach for determining a pose of an object of interest by localizing, via images acquired by a stereo infrared camera, a center of each of a plurality of similar trackers that are coupled to said objects of interest at a given relative position.

The method may comprise a step 506 of determining a spatial region 42 in which the functional element 22 is expected to be located. The spatial region 42 may be determined based on a pose of the surgical instrument 20 to which the functional element 22 is assumed to be coupled. The pose of the surgical instrument 20 can be obtained at step 512, which could in this case be performed before at least step 506. The spatial region 42 may be determined based on a predefined area or volume relative to the surgical instrument 20 in which the functional element 22 is assumed to be located if coupled to said surgical instrument 20. Based on the pose of the surgical instrument 20, the pose of the predefined area or volume can be determined and taken as the spatial region 42 in which the functional element 22 is expected to be located. Alternatively, the spatial region 42 could be determined based on user input (e.g., a user selecting said region in one or more of the at least one image comprised in the tracking image data).

The method may comprise a step 508 of selecting a region in each of one or more of the at least one image comprised in the tracking image data that corresponds to the spatial region 42 determined in step 506. This selected region may be referred to as image region or selected region. The pose of the functional element 22 may then be determined (e.g., only) based on the (e.g., content of) the selected image region(s). This may in particular reduce processing effort. For instance, the object recognition algorithm(s) could in this case be applied only to the selected image region(s) rather than to the entire image(s). In other words, the method may comprise detecting the functional element 22 in the selected image region(s) in order to determine its pose.

The method may comprise a step 510 of determining one or more possible two-dimensional poses of at least the portion of the functional element 22, for at least two (e.g., each) of the plurality of two-dimensional images comprised in the tracking image data. The tracked pose of the functional element 22 or a pose of an axis 38 of the functional element 22 may then be determined in three dimensions and/or six degrees of freedom for at least one of the points in time at which the plurality of preferably two-dimensional (non-stereo) images of the tracking image data have been acquired, in particular based on the one or more possible two-dimensional poses. Alternatively, or in addition, a length of the functional element 22 may be determined for at least one of the points in time at which the plurality of preferably two-dimensional (non-stereo) images of the tracking image data have been acquired, in particular based on the one or more possible two-dimensional poses. A single two-dimensional image may not be sufficient to accurately determine the pose and/or length of the functional element 22. In certain implementations, only a two-dimensional pose of the (e.g., center axis 38 of the) functional element may be determined based on a given two-dimensional image of the tracking image data. Two or more such two-dimensional poses can then be combined to derive the length and/or pose of the functional element 22 in three dimensions and/or six degrees of freedom. To this end, the corresponding (two-dimensional or three-dimensional) poses of the surgical instrument 20 at the different points in time associated with each of the two-dimensional images may be taken into account, and it may be assumed that the relative pose of the functional element 20 and the surgical instrument 22 do not change over the different points in time.

The method comprises a step 512 of obtaining a tracked pose of the surgical instrument 20. The tracked pose of the surgical instrument is in particular indicative of a pose of an axis 40 of the surgical instrument 20. Said axis may correspond to an axis of symmetry, a longitudinal axis and/or an insertion axis of the surgical instrument 20.

The tracked pose of the surgical instrument 20 may be determined by localizing a tracker 26 coupled to the surgical instrument 20 in a predefined relative pose. Said localizing may be performed using images acquired by a stereo camera, in particular infrared images captured by cameras 16, 18 of the tracking unit 12. Such a tracker-based localization of the surgical instrument 20 is known to those skilled in the art, so a more detailed description is avoided at this point.

Alternatively, the tracked pose of the surgical instrument 20 may be determined in a similar manner as the pose of the functional element 22. The method may thus comprise a step 514 of determining the tracked pose of the surgical instrument 20 based on the tracking image data, for example using object recognition, edge detection, outline detection and/or object classification. In this case, the tracking data should comprise at least one image of at least a portion of the surgical instrument 20 and/or of an attachment coupled to the surgical instrument in a known relative pose. In one preferred variant, the at least one image comprised in the tracking image data depicts not only (e.g., the portion of) the functional element 22, but also (e.g., the portion of or the attachment coupled to) the surgical instrument 20.

More generally speaking, in case the tracked pose of the surgical instrument 20 is determined based on the tracking image data (step 514), the tracked pose of the functional element 22 and the tracked pose of the surgical instrument 20 may both be determined based on data obtained from one and the same tracking unit 20, which preferably comprises at least one near-infrared, NIR, camera 16, 18 and a (e.g., RGB) camera 14 for acquiring the images comprised in the tracking image data.

The method may comprise a step 516 of determining a spatial region 44 in which the surgical instrument 20 is expected to be located. The spatial region 44 may be determined based on a tracker-based pose of the surgical instrument 20, which can be determined by localizing the tracker 26 coupled to said instrument 20. The spatial region 44 may be determined based on a predefined area or volume relative to the tracker 26 in which the surgical instrument 20 is assumed to be located if coupled to said tracker 26. Based on the pose of the localized tracker 26, the pose of the predefined area or volume can be determined and taken as the spatial region 44 in which the surgical instrument 20 is expected to be located. Alternatively, the spatial region 44 could be determined based on user input (e.g., a user selecting said region in one or more of the at least one image comprised in the tracking image data).

The method may comprise a step 518 of selecting a region in each of one or more of the at least one image comprised in the tracking image data that corresponds to the spatial region 44 determined in step 516. This selected region may be referred to as image region or selected region. The pose of the surgical instrument 20 may then be determined (e.g., only) based on the (e.g., content of) the selected image region(s). This may in particular reduce processing effort. For instance, an object recognition algorithm(s) could in this case be applied only to the selected image region(s) rather than to the entire image(s). In other words, the method may comprise detecting the surgical instrument 20 in the selected image region(s) in order to determine its pose.

The method comprises a step 520 of determining, based on the tracked pose of the functional element 22 and the tracked pose of the surgical instrument 20, a relative pose between the functional element 22 and the surgical instrument 20. The relative pose may be indicative of a relative position between (e.g., a predefined point of) the functional element 22 and (e.g., a predefined point of) the surgical instrument 20. The relative pose may be indicative of a relative alignment between (e.g., an axis 38 of) the functional element 22 and (e.g., an axis 40 of) the surgical instrument 20. The relative pose may be indicative of a distance and/or an angle between the axis 38 of the functional element 22 and the axis 40 of the surgical instrument.

The method may comprise a step 522 of determining a deviation between {i} the determined relative pose between the functional element 22 and the surgical instrument 20, and {ii} an ideal relative pose between the functional element 22 and the surgical instrument 20. The ideal relative pose may be referred to as a preferred relative pose. The ideal relative pose may be specific for the functional element 22, the surgical instrument 20 and/or the combination of the functional element 22 with the surgical instrument 20. The ideal relative pose may be predefined. In one example, the ideal relative pose is derived from a predefined three-dimensional ideal model. Said model may include a representation of the functional element 22 and a representation of the surgical instrument 20 with these representations exhibiting the ideal relative pose. Said model and/or ideal relative pose may be predefined by a manufacturer of either one or both of the functional element 22 and the surgical instrument 20. Alternatively, or in addition, the ideal relative pose may be defined in and/or derived from a predefined surgical plan. It is also possible for the ideal relative pose to be defined based on user input (e.g., obtained via a user input device, for example a touch display 10).

The method may comprise a step 524 of determining a relative angle between (e.g., the axis 38 of) the functional element 22 and (e.g., the axis 40 of) the surgical instrument 20. Said relative angle may be determined based on the relative pose that was determined in step 520. Step 524 may alternatively or additionally comprise determining a relative distance between the (e.g., predefined point of) functional element 22 and the (e.g., predefined point of) surgical instrument 20. Said relative distance may be determined based on the relative pose that was determined in step 520.

The method may comprise a step 526 of determining whether the functional element 22 is coupled to the surgical instrument 20. Said determination may be based on the relative pose that was determined in step 520 and/or based on the relative angle that was determined in step 524. For instance, if the relative pose indicates that the functional element 22 cannot be coupled to the surgical instrument 20 (e.g., because the two components are too far apart or the relative angle is larger than possible while coupled), it can be determined that the functional element 22 is not coupled to the surgical instrument 20. On the other hand, if the relative pose corresponds to one of a plurality of predefined possible relative poses of the functional element 22 when coupled to the surgical instrument 20, it can be determined that the functional element 22 is coupled to the surgical instrument 210.

The method may comprise a step 528 of obtaining patient tracking data indicative of a pose of a patient's body 30. The patient tracking data may be indicative of a pose of a tracker 34 that has a fixed spatial relation to the patient's body 30. The patient tracking data may be obtained from the tracking unit 12. For instance, the pose of the patient's body 30 may be determined by localizing the tracker 34 in infrared images captured by the stereo-camera 16, 18 and based on the known relative pose between the tracker 34 and the patient's body 30.

The method may comprise a step 530 of determining a distance between the functional element 20 and the patient's body. Alternatively, or in addition, a distance between the surgical instrument 22 and the patient's body may be determined. It may then be determined whether the determined distance(s) fall(s) below a predefined maximum distance. It may also be determined which of the distances falls below the predefined maximum distance.

The method comprises a step 532 of triggering output of feedback for a surgeon, the feedback indicating whether the relative pose determined in step 520 meets one or more predefined criteria.

The one or more predefined criteria may be specific for the functional element 22 and/or the surgical instrument 20 and/or a combination of the functional element 22 with the surgical instrument 20. The one or more predefined criteria may be associated with the ideal relative pose. The one or more predefined criteria may be defined in and/or derived from a predefined surgical plan. It is also possible for the one or more predefined criteria to be defined based on user input (e.g., obtained via a user input device, for example a touch display 10).

For example, the one or more predefined criteria comprise a maximum allowable angle between these two axes 38, 40. In this case, the feedback could be triggered to be output only if the angle between the two axes 38, 40 determined in step 524 is larger than the maximum allowable angle. Of course, the one or more predefined criteria may alternatively or additionally comprise a minimum allowable angle between the two axes 38, 40 or a range or set of allowable angles.

Alternatively, or in addition, the one or more predefined criteria may comprise a maximum allowable distance between the (e.g., predefined point of) functional element 22 and the (e.g., predefined point of) surgical instrument 20. In this case, the feedback could be triggered to be output only if the distance between the (e.g., predefined point of) functional element 22 and the (e.g., predefined point of) surgical instrument 20 as indicated by the determined relative pose is larger than the maximum allowable distance. Of course, the one or more predefined criteria may alternatively or additionally comprise a minimum allowable distance between the (e.g., predefined point of) functional element 22 and the (e.g., predefined point of) surgical instrument 20 or a range or set of allowable distances.

It is also possible for the one or more predefined criteria to comprise a maximum allowable deviation. In this case, the feedback could be triggered to be output only if the deviation determined in step 522 is above the maximum allowable deviation.

The one or more predefined criteria may require the functional element 22 to be coupled to the surgical instrument 22. In this case, the feedback could be triggered to be output only if it is determined in step 526 that the functional element 22 is not coupled to the surgical instrument 22.

The one or more predefined criteria may require the distance between the functional element 22 and the patient's body 30 and/or the distance between the surgical instrument 20 and the patient's body 30, as determined in step 530, to be smaller than one or more predefined maximum distances. In this case, the feedback could be triggered to be output only if at least one of the functional element 22 and the surgical instrument 20 is located within the virtual boundary 36 surrounding the patient's body 30.

The feedback that is triggered to be output may comprise auditory, visual and/or haptic feedback. In a preferred example, the feedback that is triggered to be output comprises a visualization to be displayed to a user (e.g., on the display 10). Said visualization may indicate one or more of the determined parameters such as relative pose, relative distance(s) and relative angle. The visualization may be indicative of the one or more predefined criteria, in particular the criteria that are not met. The visualization may include a representation (e.g., a rendering or a two-dimensional projection of a model) of the functional element 22 and a representation (e.g., a rendering or a two-dimensional projection of a model) of the surgical instrument 20 within the determined relative pose.

The visualization may indicate a type, name, model number, manufacturer or other information characterizing the functional element 22. The visualization may indicate a type, name, model number, manufacturer or other information characterizing the surgical instrument 20.

An exemplary visualization is shown in Fig. 1, where a two-dimensional representation 39 of the functional element 22 and a two-dimensional representation 41 of the surgical instrument 20 is shown in the relative pose as determined in step 520. The exemplary visualization also indicates a size and type of the functional element 20, in this example a screw with a length of 45 mm and a diameter of 3.5 mm. The exemplary visualization also shows a numeric value of the relative angle α between the axes 38, 40 as determined in step 524, which in this case is equal to 0.4°, and gives an indication that this angle is within the acceptable range ("OK"). In this case, the visualization is triggered to be output because the screw 22 has entered the virtual boundary 36 surrounding the patient's body, thereby informing the surgeon on the fly that the screw is correctly coupled to the screwdriver. Thus, the surgeon can proceed without having to re-adjust the screw-to-screwdriver coupling.

The technique disclosed herein will now be explained further in other words.

During spinal surgery and before placing pedicle screws or other implants like interbodies, the correct and expected functional element (e.g., screw/implant) should be assembled and secured on the surgical instrument (e.g., screwdriver/inserter). In case the functional element (e.g., pedicle screw/interbody/implant) is not properly assembled and secured, the surgeon may not be able to perform the (e.g., navigated) surgery properly and may lose time by having to verify and correct the assembly (e.g., screwdriver and screw, or any other interbody to interbody-inserter assembly). Generally speaking, the task of verifying the assembly of a screwdriver with a pedicle screw, of an implant with an implant inserter, and of a saw/drill/burr/other cutting accessory to a (e.g., navigated) power tool is cumbersome and, if done by a human, may be unreliable.

The present technique allows using RGB images in parallel of tracking (e.g., with normal exposure time) via infrared (e.g., NIR) tracking cameras 16, 18 of a tracking unit 12. In each of the RGB images (e.g., acquired by camera 14 of the same tracking unit 12), the axis 38 of the functional element (e.g., screw / implant / cutting tool) and the surgical instrument (e.g., screwdriver / inserter / power tool) can be recognized and compared. Optionally, a comparison with an ideal CAD model can be performed.

As an option, a tool center point, TCP, of the functional element (e.g., screw) and its axis 38 is recognized with one or more machine vision algorithms based on a RGB image whereas the surgical instrument itself is localized based on the images of the infrared tracking cameras 16, 18. Using multiple sets of image coordinates of the TCPs recognized in the RGB images and the according instrument localizations, 3D coordinates of the TCP and the axis 38 can be computed.

With these approaches only the RGB images and the localization information may need to be used. The technique can be performed on the fly, and a warning can be displayed to the user (e.g., step 532) in case the screw/implant/cutting element is suspected to be incorrect, misaligned or not properly secured. Additionally, for intrabodies or other implants, a correct positioning (e.g., after calibration) is hard to detect. While a screw and screwdriver may preferably build a common axis, for intrabodies the "correct" positioning may be hard to evaluate by a surgeon without performing measurements.

The technique disclosed herein may use the RGB images, the localization information of the instrument and optionally the knowledge of the ideal 3D CAD models. It may be always running in the background after screw/implant inserter or cutting tool/power tool assembly preparation and can warn the surgeon, if near the surgical site, that a misaligned/not secured screw/implant is detected. The technique is deemed to be particularly useful for spinal pedicle screw or interbody implant placement, and for bone manipulation using navigated cutting tools such as drills or saws.

Various modifications of the technique disclosed herein are possible. For example, some method steps may be omitted, replaced or shifted in sequence. Instead of a surgical screwdriver and a pedicle screw, a surgical power tool and a drill head could be made the subject of the present technique. Other modifications may be apparent to those skilled in the art.

## Claims

1. A method for supporting a surgeon in assessing a relative pose between a surgical instrument (20) and a functional element (22) configured to be coupled thereto, the method comprising:
obtaining (502) tracking image data comprising at least one image of at least a portion of a functional element (22) that is configured to be coupled to a surgical instrument (20);
determining (504), based on the tracking image data, optionally via object recognition, a tracked pose of the functional element (22);
obtaining (512) a tracked pose of the surgical instrument (20);
determining (520), based on the tracked pose of the functional element (22) and the tracked pose of the surgical instrument (20), a relative pose between the functional element (22) and the surgical instrument (20); and
triggering (532) output of feedback for a surgeon, the feedback indicating whether the relative pose meets one or more predefined criteria.

2. The method of claim 1, wherein the tracked pose of the functional element (22) is indicative of a pose of an axis (38) of the functional element (22), the tracked pose of the surgical instrument (20) is indicative of a pose of an axis (40) of the surgical instrument (20), and the one or more predefined criteria comprise a maximum allowable angle between these two axes (38, 40).

3. The method of claim 1 or 2, wherein the tracking image data comprises at least one image of:
{i} at least a portion of the surgical instrument (20); and/or
{ii} an attachment rigidly attached to the surgical instrument (20);
the method further comprising:
determining, based on the tracking image data, optionally via object recognition, the tracked pose of the surgical instrument (20).

4. The method of any one of the preceding claims, wherein the at least one image comprises an image acquired with a camera (14) configured to detect light having wavelengths perceptible by a human eye, such as an RGB image.

5. The method of any one of the preceding claims, wherein the tracked pose of the functional element (22) and the tracked pose of the surgical instrument (20) are determined based on data obtained from a tracking unit (12) comprising at least one near-infrared, NIR, camera (16; 18).

6. The method of claims 4 and 5, wherein the tracking image data is obtained from the tracking unit (12) comprising the camera (14) configured to detect light having wavelengths perceptible by a human eye.

7. The method of any one of the preceding claims, further comprising:
determining (522) a deviation between:
{i} the determined relative pose between the functional element (22) and the surgical instrument (20); and
{ii} an ideal relative pose between the functional element (22) and the surgical instrument (20), optionally derived from a predefined three-dimensional ideal model,
wherein the one or more predefined criteria comprise a maximum allowable deviation.

8. The method of any one of the preceding claims, wherein the tracking image data comprises a plurality of two-dimensional images of at least the portion of the functional element (22) that have each been acquired at different points in time, wherein (i) the tracked pose of the functional element (22) or a pose of an axis (38) of the functional element (22) at one of these points in time is determined in three dimensions based on said plurality of images and/or (i) a length of the functional element (22) is determined based on said plurality of images.

9. The method of claim 8, wherein one or more possible two-dimensional poses of at least the portion of the functional element (22) are determined for each of the plurality of two-dimensional images, and wherein
(i) the tracked pose of the functional element (22) or a pose of an axis (38) of the functional element (22), at one of the different points in time, is determined in three dimensions based on said one or more possible two-dimensional poses, and/or
(ii) the length of the functional element (22) is determined based on said one or more possible two-dimensional poses.

10. The method of any one of the preceding claims, further comprising:
determining (506; 516) a spatial region in which at least one object of interest is expected to be located;
selecting a region in each of one or more of the at least one image comprised in the tracking image data that corresponds to the spatial region of interest; and
detecting the at least one object of interest in the selected region(s).

11. The method of claim 10, wherein the at least one object of interest comprises the functional element (20) and/or the surgical instrument (22).

12. The method of any one of the preceding claims, wherein the feedback is triggered to be output if the relative pose is indicative of the functional element (22) being coupled to the surgical instrument (20) and/or wherein the one or more predefined criteria require the functional element (22) to be coupled to the surgical instrument (20).

13. The method of any one of the preceding claims, further comprising:
obtaining (528) patient tracking data indicative of a pose of a patient's body (30);
determining, based the patient tracking data and the determined pose of the functional element (22), a distance between the functional element (22) and the patient's body (30),
wherein the feedback is triggered to be output if the distance is smaller than a predefined maximum distance and/or wherein the one or more predefined criteria require the distance to be smaller than a predefined maximum distance.

14. The method of any one of the preceding claims, wherein the one or more predefined criteria are specific for the functional element (22) and/or the surgical instrument (20) and/or a combination of the functional element (22) with the surgical instrument (20).

15. The method of any one of the preceding claims, wherein one of the following conditions is met:
{i} the functional element (22) is a bone screw such as a pedicle screw and the surgical instrument (20) is a screwdriver;
{ii} the functional element (22) is an implant such as an interbody implant and the surgical instrument (20) is an implant inserter; or
{iii} the functional element (22) is a material removal unit such as a saw, a drill or a burr and the surgical instrument (20) is a power tool configured to operate the functional element (22).

16. A surgical navigation system (100) comprising at least one processor (4) configured to perform the method of any one of the preceding claims.

17. The surgical navigation system (100) of claim 16, further comprising at least one of the following entities:
a tracking unit (12) configured to acquire one of more images;
a feedback unit such as a display (10) configured to provide the feedback to a user;
the functional element (22);
the surgical instrument (20).

18. A computer program comprising instructions which, when the program is executed by at least one processor (4), cause the at least one processor (4) to carry out the method of any one of claims 1 to 15, the computer program optionally being carried by a carrier such as a data stream, a portable memory device or a non-transitory computer storage medium.

## Amended claims

### Amended claims in accordance with Rule 137(2) EPC.

1. A method for supporting a surgeon in assessing a relative pose between a surgical instrument (20) and a functional element (22) configured to be coupled thereto, the method comprising:
obtaining (502) tracking image data comprising at least one image of at least a portion of a functional element (22) that is configured to be coupled to a surgical instrument (20);
determining (504), based on the tracking image data, optionally via object recognition, a tracked pose of the functional element (22);
obtaining (512) a tracked pose of the surgical instrument (20);
determining (520), based on the tracked pose of the functional element (22) and the tracked pose of the surgical instrument (20), a relative pose between the functional element (22) and the surgical instrument (20); and
triggering (532) output of feedback for a surgeon, the feedback indicating whether the relative pose meets one or more predefined criteria.

2. The method of claim 1, wherein the tracked pose of the functional element (22) is indicative of a pose of an axis (38) of the functional element (22), the tracked pose of the surgical instrument (20) is indicative of a pose of an axis (40) of the surgical instrument (20), and the one or more predefined criteria comprise a maximum allowable angle between these two axes (38, 40).

3. The method of claim 1 or 2, wherein the tracking image data comprises at least one image of:
{i} at least a portion of the surgical instrument (20); and/or
{ii} an attachment rigidly attached to the surgical instrument (20);
the method further comprising:
determining, based on the tracking image data, optionally via object recognition, the tracked pose of the surgical instrument (20).

4. The method of any one of the preceding claims, wherein the at least one image comprises an image acquired with a camera (14) configured to detect light having wavelengths perceptible by a human eye, such as an RGB image.

5. The method of any one of the preceding claims, wherein the tracked pose of the functional element (22) and the tracked pose of the surgical instrument (20) are determined based on data obtained from a tracking unit (12) comprising at least one near-infrared, NIR, camera (16; 18).

6. The method of claims 4 and 5, wherein the tracking image data is obtained from the tracking unit (12) comprising the camera (14) configured to detect light having wavelengths perceptible by a human eye.

7. The method of any one of the preceding claims, further comprising:
determining (522) a deviation between:
{i} the determined relative pose between the functional element (22) and the surgical instrument (20); and
{ii} an ideal relative pose between the functional element (22) and the surgical instrument (20), optionally derived from a predefined three-dimensional ideal model,
wherein the one or more predefined criteria comprise a maximum allowable deviation.

8. The method of any one of the preceding claims, wherein the tracking image data comprises a plurality of two-dimensional images of at least the portion of the functional element (22) that have each been acquired at different points in time, wherein (i) the tracked pose of the functional element (22) or a pose of an axis (38) of the functional element (22) at one of these points in time is determined in three dimensions based on said plurality of images and/or (i) a length of the functional element (22) is determined based on said plurality of images.

9. The method of claim 8, wherein one or more possible two-dimensional poses of at least the portion of the functional element (22) are determined for each of the plurality of two-dimensional images, and wherein
(i) the tracked pose of the functional element (22) or a pose of an axis (38) of the functional element (22), at one of the different points in time, is determined in three dimensions based on said one or more possible two-dimensional poses, and/or
(ii) the length of the functional element (22) is determined based on said one or more possible two-dimensional poses.

10. The method of any one of the preceding claims, further comprising:
determining (506; 516) a spatial region in which at least one object of interest is expected to be located;
selecting a region in each of one or more of the at least one image comprised in the tracking image data that corresponds to the spatial region of interest; and
detecting the at least one object of interest in the selected region(s).

11. The method of claim 10, wherein the at least one object of interest comprises the functional element (20) and/or the surgical instrument (22).

12. The method of any one of the preceding claims, wherein the feedback is triggered to be output if the relative pose is indicative of the functional element (22) being coupled to the surgical instrument (20) and/or wherein the one or more predefined criteria require the functional element (22) to be coupled to the surgical instrument (20).

13. The method of any one of the preceding claims, further comprising:
obtaining (528) patient tracking data indicative of a pose of a patient's body (30);
determining, based the patient tracking data and the determined pose of the functional element (22), a distance between the functional element (22) and the patient's body (30),
wherein the feedback is triggered to be output if the distance is smaller than a predefined maximum distance and/or wherein the one or more predefined criteria require the distance to be smaller than a predefined maximum distance.

14. The method of any one of the preceding claims, wherein the one or more predefined criteria are specific for the functional element (22) and/or the surgical instrument (20) and/or a combination of the functional element (22) with the surgical instrument (20).

15. A surgical navigation system (100) comprising:
at least one processor (4) configured to perform the method of any one of the preceding claims;
the functional element (22); and
the surgical instrument (20).

16. The surgical navigation system (100) of claim 15, further comprising at least one of the following entities:
a tracking unit (12) configured to acquire one of more images;
a feedback unit such as a display (10) configured to provide the feedback to a user.

17. The surgical navigation system (100) of claim 15 or 16, wherein one of the following conditions is met:
{i} the functional element (22) is a bone screw such as a pedicle screw and the surgical instrument (20) is a screwdriver;
{ii} the functional element (22) is an implant such as an interbody implant and the surgical instrument (20) is an implant inserter; or
{iii} the functional element (22) is a material removal unit such as a saw, a drill or a burr and the surgical instrument (20) is a power tool configured to operate the functional element (22).
